# EUROPÄISCHE PATENTSCHRIFT

(11) **EP 2 735 861 B1**
(45) Veröffentlichungstag und Bekanntmachung des Hinweises auf die Patenterteilung: **07.10.2020**
(21) Anmeldenummer: 12194367.4
(22) Anmeldetag: 27.11.2012
(51) Int. Cl.: G01N 21/31

(54) **Verfahren zur Ermittlung eines Transmissionswertes**
Method for determining a transmission value
Procédé de détermination d'une valeur de transmission

(43) Veröffentlichungstag der Anmeldung: 28.05.2014
(73) Patentinhaber: Siemens Healthcare Diagnostics Products GmbH, 35041 Marburg (DE)
(72) Erfinder: Steinebach, Wolfgang, 56414 Salz (DE)

(56) Entgegenhaltungen:
- US-A- 3 973 848
- US-A- 4 857 735
- US-A1- 2004 124 377
- US-A1- 2007 253 866

## Beschreibung

Die Erfindung liegt auf dem Gebiet der in vitro-Diagnostik und betrifft ein Verfahren zur Ermittlung eines Transmissionswertes eines mit einer Frequenz gepulsten Lichtsignals durch eine Probe in einem automatischen Analysegerät. Sie betrifft weiter eine Transmissionsmessvorrichtung für ein automatisches Analysegerät, umfassend eine mit einer Frequenz gepulste Lichtquelle und einen Photodetektor mit einem nachgeschalteten A/D-Wandler.

Zahlreiche Nachweis- und Analyseverfahren zur Bestimmung physiologischer Parameter in Körperflüssigkeitsproben wie Blut, Plasma, Serum oder Urin oder in anderen biologischen Proben werden heute automatisiert in entsprechenden Analysegeräten durchgeführt.

Heutige Analysegeräte sind in der Lage, eine Vielzahl verschiedenartiger Nachweisreaktionen und Analysen mit einer Vielzahl von Proben durchzuführen. Gängige Analysegeräte, wie sie im klinischen Labor oder in Blutbanken zum Einsatz kommen, umfassen üblicherweise einen Bereich für die Zuführung von Probengefäßen, die die zu analysierenden Primärproben enthalten. Zur Einspeisung der Probengefäße in das Analysegerät ist üblicherweise ein Transportsystem vorgesehen, das die Probengefäße zunächst zu einer Probenidentifikationseinrichtung transportiert, die probenspezifische Informationen, die auf einem Probengefäß angebracht sind, erfasst und in eine Speichereinheit weiterleitet. Anschließend werden die Probengefäße zu einer Probenentnahmestation transportiert. Mit Hilfe einer Probenpipettiereinrichtung wird dort mindestens ein Aliquot der Probenflüssigkeit aus einem Probengefäß entnommen und in ein Reaktionsgefäß transferiert.

Bei den Reaktionsgefäßen handelt es sich in der Regel um Einwegküvetten, die in einem Küvettenbehälter im Analysegerät vorrätig gehalten werden und die automatisch aus dem Vorratsbehälter in definierte Aufnahmepositionen transferiert werden. Die Reagenzien, die für die Bereitstellung von verschiedenartigen, testspezifischen Reaktionsansätzen erforderlich sind, befinden sich in Reagenzbehältern, die in einer Reagenzstation aufbewahrt werden. Die Reagenzbehälter werden dem Analysegerät entweder analog der Probengefäßeinspeisung automatisch oder manuell zugeführt.

Die Reagenzstation verfügt üblicherweise über eine Kühleinheit, um eine möglichst lange Haltbarkeit der Reagenzien sicherzustellen. Mit Hilfe einer Reagenzpipettiereinrichtung, die im Übrigen häufig eine Heizvorrichtung aufweist, wird ein Aliquot eines oder mehrerer Reagenzien in ein Reaktionsgefäß transferiert, in dem sich bereits die zu untersuchende Probe befindet. Je nach Art der biochemischen Reaktion, die durch die Zugabe der Reagenzien zu einer Probe in Gang gesetzt wird, kann eine unterschiedlich lange Inkubationszeit des Reaktionsansatzes erforderlich sein. In jedem Fall wird das Reaktionsgefäß mit dem Reaktionsansatz schließlich einem Messsystem zugeführt, das eine physikalische Eigenschaft des Reaktionsansatzes misst.

Das Messergebnis wird von dem Messsystem wiederum in eine Speichereinheit weiterleitet und ausgewertet. Anschließend liefert das Analysegerät einem Benutzer über ein Ausgabemedium, wie z. B. einen Monitor, einen Drucker oder eine Netzwerkverbindung probenspezifische Messwerte.

Besonders verbreitet sind Messsysteme, die auf photometrischen (z. B. turbidimetrischen, nephelometrischen, fluorometrischen oder luminometrischen) Messprinzipien beruhen. Diese Verfahren ermöglichen den qualitativen und quantitativen Nachweis von Analyten in flüssigen Proben, ohne zusätzliche Trennschritte vorsehen zu müssen. Die Bestimmung klinisch relevanter Parameter, wie zum Beispiel der Konzentration oder der Aktivität eines Analyten erfolgt vielfach, indem ein Aliquot einer Körperflüssigkeit eines Patienten gleichzeitig oder nacheinander mit einem oder mehreren Testreagenzien im Reaktionsgefäß vermischt wird, wodurch eine biochemische Reaktion in Gang gesetzt wird, die eine messbare Veränderung einer optischen Eigenschaft des Testansatzes bewirkt. Die Photometrie untersucht und nutzt die Schwächung eines Lichtstroms bei der Transmission durch ein absorbierendes und/oder streuendes Medium. Je nach Art der ausgelösten biochemischen oder biophysikalischen Reaktion kommen unterschiedliche photometrische Messverfahren zum Einsatz, die die Messung eines trüben flüssigen Testansatzes ermöglichen.

Bei turbidimetrischen Verfahren wird die Trübung beziehungsweise die optische Dichte einer Lösung oder Dispersion (Suspension) anhand der Lichtschwächung oder Extinktion eines direkt durch die Dispersion (Suspension) hindurch tretenden Lichtstrahls gemessen. Die Transmission des Lichtstrahls wird über einen Photodetektor erfasst, der eine der Stärke des durch die Probe transmittierten Lichtsignals zugeordnete Spannungskurve erzeugt.

Zur Verbesserung des Störabstandes und zur Unterdrückung von niederfrequentem Rauschen kommt bei Photodetektoren häufig ein Lock-In-Verstärker zum Einsatz. Hierbei wird ein moduliertes Messsignal, d. h. ein durch die Probe gesendetes und vom Photodetektor empfangenes gepulstes Lichtsignal, über einen Bandpass einem Multiplizierer zugeführt, welcher das Messsignal mit einem phasenverkoppelten Referenzsignal gleicher Frequenz moduliert und nach einer Tiefpassfilterung ein Ausgangssignal erzeugt, welches der Amplitude des modulierten Eingangssignals proportional ist.

Ein derartiger Lock-In-Verstärker kann auch in digitalen Systemen wie z. B. FPGAs (Field Programmable Gate Array), d. h. integrierten Schaltkreisen realisiert werden. Dabei wird das Messsignal zunächst einem A/D-Wandler zugeführt, der eine Mehrzahl von Abtastwerten der Spannungskurve aus dem Photometer ermittelt. Dabei werden die gleichen Blockstrukturen einer analogen Schaltung durch digitale Blöcke realisiert. Es müssen dazu jedoch diverse Einheiten zur Signalverarbeitung wie Bandpassfilter, Multiplizierer und Tiefpass erzeugt werden. Daher wird das System vergleichsweise komplex, und es wird eine erhöhte Menge von Logikelementen benötigt.

Es ist daher Aufgabe der Erfindung, ein Verfahren zur Ermittlung eines Transmissionswertes sowie eine Transmissionsmessvorrichtung der eingangs genannten Art anzugeben, die das Prinzip und die Vorteile des Lock-In-Verstärkers in einer digitalen Umsetzung aufweisen, ohne einen erhöhten Bedarf an Logikelementen zu verursachen.

US2007/253866 A1 offenbart ein Verfahren bzw. Transmissionsmessvorrichtung zur Bestimmung der Konzentration oder Aktivität mindestens eines Analyten in einer Körperflüssigkeitsprobe.

US4857735 A offenbart ein Verfahren zur spektroskopischen Analyse von Körperflüssigkeitsproben, wobei eine mit einer Frequenz gepulste Lichtquelle verwendet wird.

US2004/124377 A1 offenbart eine digital gesteuerte pyroelektrische Signalabtastschaltung, die ein Wechselstromsignal von einem pyroelektrischen Infrarotsensor verarbeitet.

Bezüglich des Verfahrens wird die Aufgabe erfindungsgemäß gelöst, indem das Verfahren die folgenden Schritte umfasst:
a) Erzeugung einer der Stärke des durch die Probe transmittierten Lichtsignals zugeordneten Spannungskurve,
b) Ermittlung einer Mehrzahl von Abtastwerten der Spannungskurve,
c) Ermittlung eines einem Lichtpuls zugeordneten ersten Spannungswertes aus einer Anzahl von Abtastwerten und Ermittlung eines einer Dunkelzeit zwischen zwei Lichtpulsen zugeordneten zweiten Spannungswertes aus einer Anzahl von Abtastwerten, wobei die Abtastwerte zur Ermittlung eines einer Dunkelzeit zwischen zwei Lichtpulsen zugeordneten zweiten Spannungswertes gegenüber den Abtastwerten zur Ermittlung eines einem Lichtpuls zugeordneten ersten Spannungswertes um ein ganzzahlig Vielfaches einer der Frequenz des gepulsten Lichtsignals entsprechenden halben Periode versetzt sind, und
d) Subtraktion des zweiten Spannungswertes vom ersten Spannungswert.

Die in Schritt d) erhaltene Differenz ist proportional zum Transmissionswert. Erfindungsgemäß wird in dem Verfahren anhand des Transmissionswertes die Konzentration oder Aktivität mindestens eines Analyten in einer Körperflüssigkeitsprobe bestimmt.

Die Erfindung geht dabei von der Überlegung aus, dass beim Lock-In-Verfahren im Falle einer einfach geschalteten Signal- und Referenzquelle letztlich im Multiplizierer eine einfache Multiplikation der aus dem Nutzsignal ermittelten Spannungskurve mit den Faktoren +1 und -1 in Abhängigkeit von der Phasenlage des rechteckförmig modulierten Signales erfolgt. Signalanteile mit Nutzsignalinhalt, d. h. solche aus Lichtpulsen werden dabei mit +1, Signalanteile ohne Nutzsignal, d. h. von Dunkelzeiten zwischen den Lichtpulsen mit -1 multipliziert. Wenn beide Komponenten niederfrequente Rauschanteile oder Gleichanteile enthalten, werden sie nach der Multiplikation durch Tiefpassfilterung entfernt. Die Tiefpassfilterung wirkt dabei als Integrierglied über mehrere Signalperioden, so dass letztlich eine Summierung über die negativen signalfreien Rauschanteile und die positiven Signalanteile stattfindet. Dieses Ergebnis wird auch erreicht, wenn ein einer Dunkelzeit entsprechender Spannungswert direkt von einem Lichtpuls entsprechenden Spannungswert subtrahiert wird. Somit ist keine Multipliziereinheit für die Signalverarbeitung mehr erforderlich.

Für den ersten Spannungswert verwendete Abtastwerte und für den zweiten Spannungswert verwendete Abtastwerte sind um ein ganzzahlig Vielfaches einer der Frequenz des gepulsten Lichtsignals entsprechenden halben Periode versetzt und damit in der Phasenlage fest korreliert. Dies ermöglicht eine kontinuierliche Abtastung des Signals bzw. der dem Signal zugeordneten Spannungskurve in gleichen Abständen, wobei durch die Korrelation mit der Frequenz und Phasenlage der Lichtpulse die Abtastwerte gezielt für den Signal-plus-Rauschanteil sowie für den reinen Rauschanteil ausgewählt werden. Dadurch wird ein kontinuierlich rauscharmes Ergebnissignal erzeugt.

In vorteilhafter Ausgestaltung werden in Schritt c), d.h. bei der Ermittlung der Spannungswerte, die einem Lichtpuls beziehungsweise einer Dunkelzeit zugeordnet sind, Abtastwerte in einem vorgegebenen Übergangsbereich zwischen Lichtpuls und Dunkelzeit verworfen. Dies betrifft Abtastwerte, die in die steigende oder fallende Flanke des Signals fallen und somit keine stabilen Signalwerte ergeben. Eine Verwerfung derartiger Signale verbessert die Genauigkeit des Ergebnisses.

Vorteilhafterweise folgt die jeweilige Dunkelzeit, deren Abtastwert für den zweiten Spannungswert herangezogen wird, auf den jeweiligen Lichtpuls, dessen Abtastwert für den ersten Spannungswert herangezogen wird, unmittelbar oder geht dem jeweiligen Lichtpuls unmittelbar voran. Einerseits erfordert eine Subtraktion aufeinanderfolgender Lichtpulse und Dunkelzeiten den geringsten technischen Aufwand, andererseits sind aufeinanderfolgende Lichtpulse und Dunkelzeiten am ehesten den gleichen Störquellen ausgesetzt, so dass eine Subtraktion die Störquellen optimal herausfiltert, was die Ergebnisqualität erhöht.

In einer weiteren vorteilhaften Ausgestaltung werden die Schritte b), c) und d) zyklisch wiederholt, und über die in Schritt d) ermittelten Werte wird ein Mittelwert gebildet. Die Mittelwertbildung filtert dabei weitere Störanteile aus dem Ergebnis, so dass eine noch bessere Qualität des Ergebnisses erreicht wird.

Insbesondere betrifft die Erfindung ein Verfahren zur Bestimmung der Konzentration oder Aktivität mindestens eines Analyten in einer Körperflüssigkeitsprobe, vorzugsweise in einer Körperflüssigkeitsprobe aus der Gruppe Vollblut, Blutplasma, Serum, Liquor oder Urin.

Eine erfindungsgemäße Transmissionsmessvorrichtung umfasst Mittel zum Ausführen des beschriebenen Verfahrens.

Bezüglich der Transmissionsmessvorrichtung wird die Aufgabe gelöst, indem der A/D-Wandler eine Abtastfrequenz aufweist, die einem geraden Vielfachen der Frequenz des gepulsten Lichtsignals entspricht, und die Transmissionsmessvorrichtung einen dem A/D-Wandler nachgeschalteten integrierten Schaltkreis aufweist, der dafür ausgebildet ist, Abtastwerte des A/D-Wandlers auf Basis der Frequenz und Phasenlage einem Lichtpuls oder einer Dunkelzeit zwischen zwei Lichtpulsen zuzuordnen, die Lichtpulsen zugeordneten Abtastwerte oder die Dunkelzeiten zugeordneten Abtastwerte um eine der Frequenz entsprechende halbe Periode zu verzögern und dadurch entstehende zeitgleiche Abtastwerte voneinander zu subtrahieren.

Die Erfindung betrifft daher ferner eine Transmissionsmessvorrichtung zur Bestimmung der Konzentration oder Aktivität mindestens eines Analyten in einer Körperflüssigkeitsprobe und für ein automatisches Analysegerät, umfassend eine mit einer Frequenz gepulste Lichtquelle, einen Photodetektor mit einem nachgeschalteten A/D-Wandler mit einer Abtastfrequenz, die einem geraden Vielfachen der Frequenz der gepulsten Lichtquelle entspricht, sowie einen dem A/D-Wandler nachgeschalteten integrierten Schaltkreis, der dafür ausgebildet ist,
i) Abtastwerte des A/D-Wandlers auf Basis der Frequenz der gepulsten Lichtquelle und Phasenlage einem Lichtpuls oder einer Dunkelzeit zwischen zwei Lichtpulsen zuzuordnen, und
ii) die den Lichtpulsen zugeordneten Abtastwerte oder die den Dunkelzeiten zugeordneten Abtastwerte um ein ganzzahlig Vielfaches einer der Frequenz der gepulsten Lichtquelle entsprechenden halben Periode zu verzögern, und
iii) dadurch entstehende zeitgleiche Abtastwerte voneinander zu subtrahieren.

Vorteilhafterweise hat die Abtastfrequenz zumindest den vierfachen Wert der Frequenz der gepulsten Lichtquelle. Hierdurch wird eine Überabtastung erreicht, wobei jedem Lichtpuls und jeder Dunkelzeit bei einer vierfachen Abtastfrequenz bereits jeweils zwei Abtastwerte zugeordnet werden können.

In weiterer vorteilhafter Ausgestaltung ist der integrierte Schaltkreis dafür ausgebildet, einen Mittelwert über die subtrahierten zeitgleichen Abtastwerte zu bilden. Durch die Erhöhung der digitalen Auflösung insbesondere in Verbindung mit der Mittelwertbildung wird die Qualität des Ergebnissignals weiter verbessert.

In weiterer vorteilhafter Ausgestaltung enthält der integrierte Schaltkreis ein Field Programmable Gate Array (FPGA). Dies ist insbesondere deshalb vorteilhaft, da in einigen bekannten automatischen Analysegeräten solche FPGAs zur Abarbeitung anderer Aufgaben, wie z.B. zur Realisierung eines Prozessorsystems, zur Auswertung von Messsignalen oder zur Steuerung von Motoren oder verschiedener Aktoren eingesetzt werden und deshalb bereits in dem Gerät enthalten sind. Ist ein FPGA also bereits vorhanden, können leicht weitere Funktionen, wie das hier beschriebene Verfahren, implementiert werden, ohne dass eine Neuentwicklung einer Mikroprozessorschaltung notwendig wird. Dies erhöht die Flexibilität gegenüber Änderungen bei gewünschten neuen Eigenschaften eines automatischen Analysegeräts. Desweiteren ist es vorteilhaft, dass in FPGAs komplexe Funktionen in deutlich höherer Geschwindigkeit als in Mikroprozessoren realisiert werden können.

Die mit der Erfindung erzielten Vorteile bestehen insbesondere darin, dass durch die Nutzung einer Zeitverschiebung, Subtraktion und Mittelwertbildung bei der Verminderung von Rauschanteilen im Photodetektorsignal einer Transmissionsmessvorrichtung eine Vereinfachung der Struktur ohne komplexe Signalprozessierungsblöcke wie z. B. Filter oder Multiplizierer möglich wird. Dadurch kann auf kostengünstige Logik, z. B. einfache algebraische Operationen zurückgegriffen werden. Nicht nur im Vergleich zu DSP (Digital Signal Processing)-Design, auch im Vergleich zu analoger Technik ist die Lösung kostengünstiger. Es sind keine komplexen Abgleiche wie z. B. Verstärkung, Phasenlage oder Filtercharakteristik notwendig. Eine Drift des Signals wie bei analogen Verfahren ist ebenfalls nicht zu erwarten.

### FIGURENBESCHREIBUNG

Die Erfindung wird anhand einer Zeichnung näher erläutert. Darin zeigen:
- FIG 1: eine Transmissionsmessvorrichtung mit einem Lock-In-Verstärker nach dem Stand der Technik,
- FIG 2: die prinzipielle Funktionsweise des bekannten Lock-In-Verstärkers aus FIG 1 im Falle eines einfach geschalteten Signals,
- FIG 3: eine mögliche Umsetzung der Funktionsweise nach FIG 2, und
- FIG 4: einen Ausschnitt einer erfindungsgemäßen Transmissionsmessvorrichtung mit einer korrelierten Überabtastung.

Gleiche Teile sind in allen Figuren mit denselben Bezugszeichen versehen.

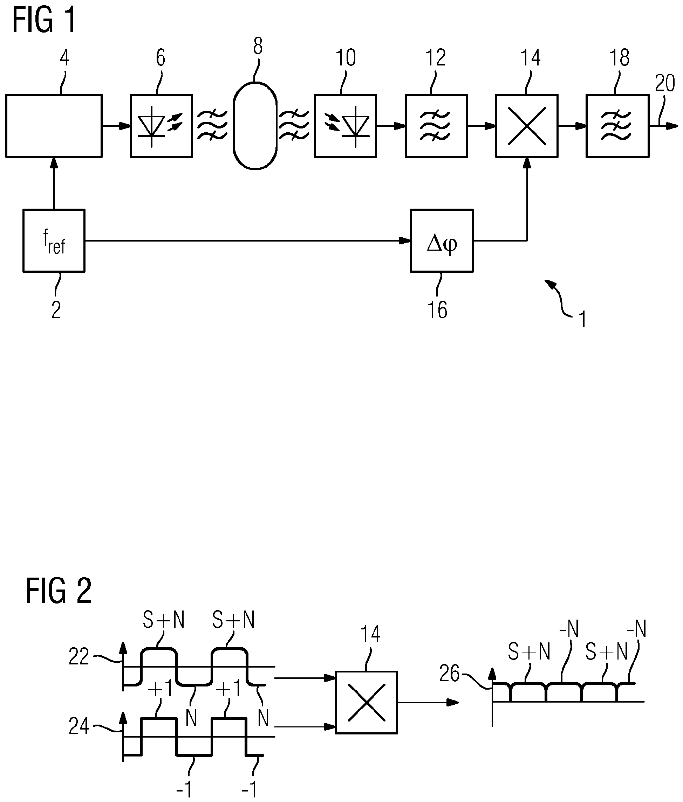

Die Transmissionsmessvorrichtung 1 nach der FIG 1 zeigt die Funktionsweise eines üblichen Lock-In-Verstärkers. Sie weist zunächst einen Frequenzgenerator 2 auf, dem ein Modulator 4 nachgeschaltet ist. Der Modulator 4 moduliert das typischerweise um einen Nullwert oszillierende Signal des Frequenzgenerators 2 derart, dass eine gleichmäßige Einschalt- und Ausschaltoszillation für eine Lichtquelle 6, hier eine Leuchtdiode, erreicht wird. Die Lichtquelle 6 wird somit mit der Frequenz des Frequenzgenerators 2 gepulst. Alternativ könnte auch eine kontinuierliche Lichtquelle 6 mit einem Chopper-Rad mit entsprechender Umlauffrequenz zur Anwendung kommen.

Die Lichtquelle 6 durchleuchtet eine Probe 8, im vorliegenden Fall eine Blutprobe in einem nicht näher dargestellten automatischen Analysegerät. Die Blutprobe wird dabei einer chemischen Reaktion unterzogen, während derer die Absorption des Lichtes der Lichtquelle 6 mittels einer Transmissionsmessung ermittelt werden soll. Das transmittierte Licht wird von einem Photodetektor 10 erfasst.

Dem Photodetektor 10 ist zunächst ein Bandpassfilter 12 nachgeschaltet, dessen Übertragungsfunktion ein Maximum im Bereich der Frequenz des Frequenzgenerators 2 hat. Das Bandpassfilter 12 filtert einerseits störende Signalanteile außerhalb der Pulsfrequenz der Lichtquelle 6 heraus, andererseits erzeugt es aus dem zwischen Null und einem Maximalwert oszillierenden Signal des Photodetektors 10 wieder ein um Null oszillierendes Signal. Dieses wird in einem Multiplizierer 14 mit dem Signal aus dem Frequenzgenerator 2, welches in einem Phasenverschieber 16 phasenverschoben wird, multipliziert. Die Phasenverschiebung wird dabei derart eingestellt, dass ein maximales Ausgangssignal am Multiplizierer 14 erreicht wird.

Das Ausgangssignal wird schließlich einem Tiefpassfilter 18 zugeführt, das letztlich als Integrierer wirkt und ein Gleichspannungssignal als Ergebnissignal 20 ausgibt. Das Ergebnissignal 20 ist proportional zur Transmission durch die Probe 8 und von Störeinflüssen weitgehend befreit. Das in FIG 1 gezeigte Verfahren wird insbesondere verwendet, um die Empfindlichkeit für niedrige Signalpegel zu erhöhen. Einflüsse von niederfrequentem Rauschen (z. B. 1/f-Rauschen) und Fremdlicht (Tageslicht, künstliche Beleuchtung) werden gemindert.

Eine Umsetzung der in FIG 1 gezeigten Transmissionsmessvorrichtung 1 in digitalen Systemen wie z. B. FPGAs ist grundsätzlich möglich, benötigt jedoch eine vergleichsweise komplexe digitale Umsetzung der DSP-Module wie Bandpassfilter 12, Multiplizierer 14 und Tiefpassfilter 18.

FIG 2 zeigt eine vereinfachte Darstellung der Funktionsweise des Multiplizierers 14 im Falle eines einfach geschalteten Signals wie z. B. einer gepulsten Lichtquelle 6 in einem automatischen Analysegerät. Eine Phasenverschiebung ist typischerweise nicht zu erwarten. Das Photodetektorsignal 22 wird mit dem zwischen -1 und +1 oszillierenden Rechtecksignal 24 aus dem Frequenzgenerator 2 multipliziert. Da die +1-Bereiche stets den Lichtpulsen der Lichtquelle 6 entsprechen, werden somit Signalanteile mit Nutzsignal S stets mit +1 multipliziert, während Signale aus Dunkelzeiten, die nur Rauschen N enthalten, mit -1 multipliziert werden. Dadurch ergibt sich das in FIG 2 dargestellte Ausgangssignal 26 mit alternierenden S+N und -N-Bereichen. Wenn beide Komponenten niederfrequente Rauschanteile oder Gleichanteile enthalten, werden diese nach der Multiplikation durch Tiefpassfilterung entfernt.

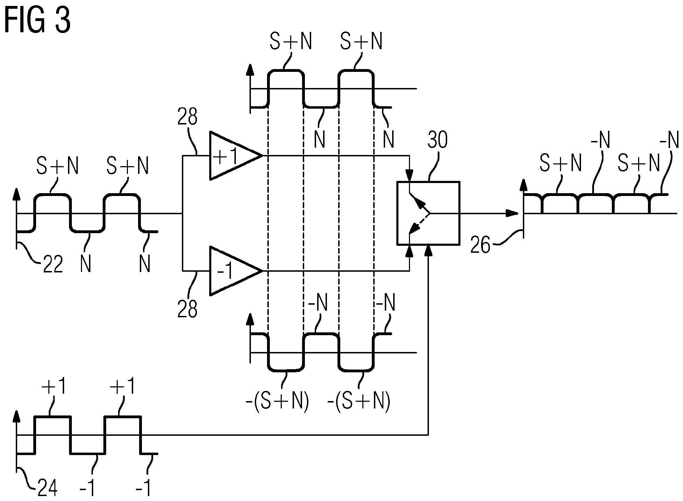

Faktisch lässt sich das in FIG 2 gezeigte Prinzip durch die in FIG 3 gezeigte Schaltung umsetzen. Das Photodetektorsignal wird in zwei Äste 28 aufgespalten, von denen einer mit -1 multipliziert wird. Die entsprechenden Signale sind zu jedem Ast 28 in FIG 3 dargestellt. Das Rechtecksignal 24 steuert einen Schalter 30, der mit der Frequenz des Rechtecksignals 24 zwischen den beiden Ästen 28 schaltet. Dadurch entsteht das bereits in FIG 2 gezeigte Ausgangssignal 26.

Eine nachfolgende Tiefpassfilterung ist im einfachen, in FIG 2 und 3 dargestellten Fall als Mittelwertbildung über mehrere Perioden realisierbar. Dabei wird somit letztlich eine Subtraktion des signallosen Rauschens N vom Nutzsignal mit Rauschanteil S+N vorgenommen. Es ergibt sich als Ausgangssignal S=(S+N)-N.

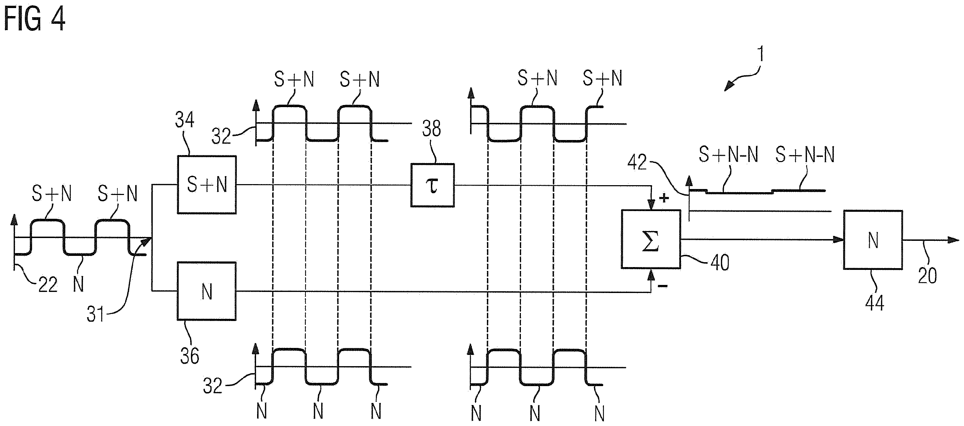

Dieses Ergebnis wird aber auch erreicht, wenn der signalfreie Rauschanteil N direkt vom vorhergehenden oder folgenden Signalanteil S subtrahiert wird. Dadurch ist kein Multiplizierer 14 mehr erforderlich. Eine nach diesem Prinzip arbeitende Transmissionsmessvorrichtung 1 ist in FIG 4 ausschnittsweise dargestellt. Die In FIG 4 dargestellte Schaltung ist auf einem integrierten Schaltkreis, nämlich einem FPGA implementiert.

Das Photodetektorsignal 22 wird einem A/D-Wandler 31 zugeführt, der einerseits eine Abtastung der S+N-Bereiche, als auch der N-Bereiche durchführt. Im Ausführungsbeispiel ist der A/D-Wandler 31 als 18-Bit-A/D-Wandler ausgeführt. Die Frequenz der Lichtquelle beträgt 45 kHz, der A/D-Wandler ist für eine 20-fache Abtastfrequenz ausgelegt, so dass jeweils zehn Abtastwerte 32 pro Periode als S+N-Abtastwerte 34 und die N-Abtastwerte 36 gespeichert und verarbeitet werden. Da die Frequenz des A/D-Wandlers 31 mit der Frequenz der Lichtquelle 6 korreliert ist, sind die Abtastwerte 32 unmittelbar einem Lichtpuls oder einer Dunkelzeit zuordbar. Abtastwerte 32, die auf die steigende oder fallende Flanke fallen, werden ignoriert bzw. verworfen.

Durch die Überabtastung einer Lock-In-Periode von zwanzig Signalwerten im Ausführungsbeispiel unter Verwendung von jeweils vier Signalanteilwerten und vier Rauschanteilwerten wird das Ergebnis der Signalwertberechnung bereits um den Faktor vier erhöht, was zwei Bit digitaler Auflösung entspricht. Unkorreliertes hochfrequentes Rauschen wird um den Faktor Wurzel vier reduziert, was zu einer ersten Verbesserung des Störabstandes führt.

Die S+N-Abtastwerte 34 werden in einem Verzögerungsglied 38 um eine einer halben Periode der Frequenz der Lichtquelle 6 entsprechende Zahl von Abtastwerten 32 verzögert. In einem Subtrahierglied 40 werden zeitgleiche, verzögerte S+N-Abtastwerte 34 und N-Abtastwerte 36 voneinander subtrahiert. Hierdurch kann insbesondere auch eine dem Photodetektor 10 nachgeschaltete Bandpassfilterung entfallen. Das Ausgangssignal 42 des Subtrahiergliedes 40 wird einer einfachen, über N Perioden einstellbaren Mittelwertbildung 44 zugeführt. Im Ausführungsbeispiel wird über 1024 Lock-In-Perioden, d. h. Perioden der Lichtquelle 6 gemittelt.

Durch die dargestellte Schaltung ergibt sich für das Ergebnissignal 20 eine entsprechende Erhöhung der Auflösung um 10 Bit und für unkorreliertes Rauschen eine weitere Verbesserung des Störabstandes von theoretisch Wurzel 1024 = 32 (oder 30 dB, 5 Bit digital). Unter realen Bedingungen ist allerdings nicht von unkorreliertem Rauschen auszugehen, so dass der reale Auflösungsgewinn niedriger ausfällt. Messungen haben eine äquivalente Auflösung von ca. 22 bis 23 Bit im Ausführungsbeispiel ergeben, was einem Störabstand von mehr als 130 dB entspricht. Die Empfindlichkeit gegen von außen auftreffendes Fremdlicht ist dabei sehr gering.

### BEZUGSZEICHENLISTE

- 1: Transmissionsmessvorrichtung
- 2: Frequenzgenerator
- 4: Modulator
- 6: Lichtquelle
- 8: Probe
- 10: Photodetektor
- 12: Bandpassfilter
- 14: Multiplizierer
- 16: Phasenverschieber
- 18: Tiefpassfilter
- 20: Ergebnissignal
- 22: Photodetektorsignal
- 24: Rechtecksignal
- 26: Ausgangssignal
- 28: Äste
- 30: Schalter
- 31: A/D-Wandler
- 32: Abtastwerte
- 34: S+N-Abtastwerte
- 36: N-Abtastwerte
- 38: Verzögerungsglied
- 40: Subtrahierglied
- 42: Ausgangssignal
- 44: Mittelwertbildung

- S: Nutzsignal
- N: Rauschen

## Patentansprüche

1. Verfahren zur Bestimmung der Konzentration oder Aktivität mindestens eines Analyten in einer Körperflüssigkeitsprobe anhand mindestens eines Transmissionswertes eines mit einer Frequenz gepulsten Lichtsignals durch eine Probe (8) in einem automatischen Analysegerät, wobei der Transmissionswert mit einem Verfahren mit den folgenden Schritten ermittelt wird:
a) Erzeugung einer der Stärke des durch die Probe (8) transmittierten Lichtsignals zugeordneten Spannungskurve (22),
b) Ermittlung einer Mehrzahl von Abtastwerten (32, 34, 36) der Spannungskurve (22),
c) Ermittlung eines einem Lichtpuls zugeordneten ersten Spannungswertes aus einer Anzahl von Abtastwerten (34) und Ermittlung eines einer Dunkelzeit zwischen zwei Lichtpulsen zugeordneten zweiten Spannungswertes aus einer Anzahl von Abtastwerten (36), wobei die Abtastwerte (36) zur Ermittlung eines einer Dunkelzeit zwischen zwei Lichtpulsen zugeordneten zweiten Spannungswertes gegenüber den Abtastwerten (34) zur Ermittlung eines einem Lichtpuls zugeordneten ersten Spannungswertes um ein ganzzahlig Vielfaches einer der Frequenz des gepulsten Lichtsignals entsprechenden halben Periode versetzt sind, und
d) Subtraktion des zweiten Spannungswertes vom ersten Spannungswert,
wobei die in Schritt d) erhaltene Differenz proportional zum Transmissionswert ist.

2. Verfahren nach Anspruch 1, bei dem in Schritt c) Abtastwerte (32) in einem vorgegebenen Übergangsbereich zwischen Lichtpuls und Dunkelzeit verworfen werden.

3. Verfahren nach Anspruch 1 oder 2, bei dem die jeweilige Dunkelzeit auf den jeweiligen Lichtpuls unmittelbar folgt oder dem jeweiligen Lichtpuls unmittelbar vorangeht.

4. Verfahren nach einem der vorhergehenden Ansprüche, bei dem die Schritte b), c) und d) zyklisch wiederholt werden und über die in Schritt d) ermittelten Transmissionswerte ein Mittelwert gebildet wird.

5. Verfahren nach Anspruch 1, wobei die Konzentration oder Aktivität des Analyten in Vollblut, Blutplasma, Serum, Liquor oder Urin bestimmt wird.

6. Transmissionsmessvorrichtung (1) zur Bestimmung der Konzentration oder Aktivität mindestens eines Analyten in einer Körperflüssigkeitsprobe und für ein automatisches Analysegerät, umfassend eine mit einer Frequenz gepulste Lichtquelle (6), einen Photodetektor (10) mit einem nachgeschalteten A/D-Wandler (31) mit einer Abtastfrequenz, die einem geraden Vielfachen der Frequenz der gepulsten Lichtquelle entspricht, sowie einen dem A/D-Wandler (31) nachgeschalteten integrierten Schaltkreis, der dafür ausgebildet ist,
i) Abtastwerte des A/D-Wandlers (31) auf Basis der Frequenz der gepulsten Lichtquelle und Phasenlage einem Lichtpuls oder einer Dunkelzeit zwischen zwei Lichtpulsen zuzuordnen, und
ii) die den Lichtpulsen zugeordneten Abtastwerte (34) oder die den Dunkelzeiten zugeordneten Abtastwerte (36) um ein ganzzahlig Vielfaches einer der Frequenz der gepulsten Lichtquelle entsprechenden halben Periode zu verzögern, und
iii) dadurch entstehende zeitgleiche Abtastwerte (32) voneinander zu subtrahieren.

7. Transmissionsmessvorrichtung (1) nach Anspruch 6, bei der die Abtastfrequenz zumindest den vierfachen Wert der Frequenz der gepulsten Lichtquelle (6) hat.

8. Transmissionsmessvorrichtung (1) nach Anspruch 6 oder 7, bei der der integrierte Schaltkreis dafür ausgebildet ist, einen Mittelwert über die subtrahierten zeitgleichen Abtastwerte (32) zu bilden.

9. Transmissionsmessvorrichtung (1) nach einem der Ansprüche 6 bis 8, bei der der integrierte Schaltkreis ein Field Programmable Gate Array (FPGA) enthält.

10. Automatisches Analysegerät mit einer Transmissionsmessvorrichtung (1) nach einem der Ansprüche 6 bis 9.

## Claims

1. Method for determining the concentration or activity of at least one analyte in a body fluid sample on the basis of at least one transmission value for a light signal that is pulsed at a frequency through a specimen (8) in an automatic analysis appliance, wherein the transmission value is ascertained using a method having the following steps:
a) producing a voltage curve (22) associated with the intensity of the light signal transmitted through the specimen (8),
b) ascertaining a plurality of sample values (32, 34, 36) for the voltage curve (22),
c) ascertaining a first voltage value associated with a light pulse from a number of sample values (34) and ascertaining a second voltage value associated with a dark time between two light pulses from a number of sample values (36), wherein the sample values (36) for ascertaining a second voltage value associated with a dark time between two light pulses are offset from the sample values (34) for ascertaining a first voltage value associated with a light pulse by an integer multiple of a half-period corresponding to the frequency of the pulsed light signal, and
d) subtracting the second voltage value from the first voltage value,
wherein the difference obtained in step d) is proportional to the transmission value.

2. Method according to Claim 1, in which sample values (32) in a prescribed transition region between light pulse and dark time are rejected in step c).

3. Method according to Claim 1 or 2, in which the respective dark time follows the respective light pulse directly or precedes the respective light pulse directly.

4. Method according to one of the preceding claims, in which steps b), c) and d) are cyclically repeated and a mean value is formed over the transmission values ascertained in step d).

5. Method according to Claim 1, wherein the concentration or activity of the analyte is determined in whole blood, blood plasma, serum, liquor or urine.

6. Transmission measurement apparatus (1) for determining the concentration or activity of at least one analyte in a body fluid sample and for an automatic analysis appliance, comprising a light source (6) that is pulsed at a frequency, a photodetector (10) having a downstream A/D converter (31) with a sampling frequency that corresponds to an even multiple of the frequency of the pulsed light source, and also an integrated circuit that is connected downstream of the A/D converter (31) and that is designed to
i) associate sample values from the A/D converter (31) with a light pulse or with a dark time between two light pulses on the basis of the frequency of the pulsed light source and phase, and
ii) delay the sample values (34) associated with the light pulses or the sample values (36) associated with the dark times by an integer multiple of a half-period corresponding to the frequency of the pulsed light source, and
iii) subtract resultant simultaneous sample values (32) from one another.

7. Transmission measurement apparatus (1) according to Claim 6, in which the sampling frequency has at least four times the value of the frequency of the pulsed light source (6).

8. Transmission measurement apparatus (1) according to Claim 6 or 7, in which the integrated circuit is designed to form a mean value over the subtracted simultaneous sample values (32).

9. Transmission measurement apparatus (1) according to one of Claims 6 to 8, in which the integrated circuit contains a field programmable gate array (FGPA).

10. Automatic analysis appliance having a transmission measurement apparatus (1) according to one of Claims 6 to 9.

## Revendications

1. Procédé de détermination de la concentration ou de l'activité d'au moins un analyte dans un échantillon de liquide corporel à l'aide d'au moins une valeur de transmission d'un signal lumineux pulsé à une fréquence à travers un échantillon (8) dans un appareil d'analyse automatique, dans lequel on détermine la valeur de transmission par un procédé ayant les stades suivants :
a) production d'une courbe (22) de tension associée à l'intensité du signal lumineux transmis à travers l'échantillon (8),
b) détermination d'une pluralité de valeurs (32, 34, 36) d'échantillonnage de la courbe (22) de tension,
c) détermination d'une première valeur de tension associée à une impulsion lumineuse parmi un nombre de valeurs (34) d'échantillonnage et détermination d'une deuxième valeur de tension, associée à un temps d'obscurité entre deux impulsions lumineuses, parmi un nombre de valeurs (36) d'échantillonnage, les valeurs (36) d'échantillonnage étant, pour la détermination d'une deuxième valeur de tension, associée à un temps d'obscurité entre deux impulsions lumineuses, décalées par rapport aux valeurs (34) d'échantillonnage pour la détermination d'une première valeur de tension associée à une impulsion lumineuse, d'un multiple en nombre entier d'une demi-période correspondant à la fréquence du signal lumineux pulsé, et
d) soustraction de la deuxième valeur de tension de la première valeur de tension,
dans lequel la différence obtenue au stade d) est proportionnelle à la valeur de transmission.

2. Procédé suivant la revendication 1, dans lequel, dans le stade c), on rejette des valeurs (32) d'échantillonnage dans une plage de transition donnée à l'avance entre impulsion lumineuse et temps d'obscurité.

3. Procédé suivant la revendication 1 ou 2, dans lequel le temps d'obscurité respectif suit directement l'impulsion lumineuse respective ou précède directement l'impulsion lumineuse respective.

4. Procédé suivant l'une des revendications précédentes, dans lequel on répète cycliquement les stades b), c) et d) et on forme une valeur moyenne par les valeurs de transmission déterminées au stade d).

5. Procédé suivant la revendication 1, dans lequel on détermine la concentration ou l'activité de l'analyte dans du sang total, du plasma sanguin, du sérum, du liquide céphalorachidien ou de l'urine.

6. Système (1) de mesure de transmission pour la détermination de la concentration ou de l'activité d'au moins un analyte dans un échantillon de liquide corporel et pour un appareil d'analyse automatique, comprenant une source (6) lumineuse pulsée à une fréquence, un photodétecteur (10) ayant un convertisseur (31) analogique/numérique en aval, ayant une fréquence d'échantillonnage qui correspond à un multiple pair de la fréquence de la source lumineuse pulsée, ainsi qu'un circuit intégré en aval du convertisseur (31) analogique/numérique, qui est constitué pour
i) associer des valeurs d'échantillonnage du convertisseur (31) analogique/numérique sur la base de la fréquence de la source lumineuse pulsée et de la position en phase d'une impulsion lumineuse ou d'un temps d'obscurité entre deux impulsions lumineuses, et
ii) retarder les valeurs (36) d'échantillonnage, associées aux valeurs (34) d'échantillonnage associées aux impulsions lumineuses ou associées au temps d'obscurité, d'un multiple en nombre entier d'une demi-période correspondant à la fréquence de la source lumineuse puisée, et
iii) soustraire entre elles des valeurs (32) d'échantillonnage créées en même temps.

7. Système (1) de mesure de transmission suivant la revendication 6, dans lequel la fréquence d'échantillonnage est au moins le quart de la fréquence de la source (6) lumineuse puisée.

8. Système (1) de mesure de transmission suivant la revendication 6 ou 7, dans lequel le circuit intégré est constitué pour former une valeur moyenne sur les valeurs (32) d'échantillonnage soustraites obtenues en même temps.

9. Système (1) de mesure de transmission suivant l'une des revendications 6 à 8, dans lequel le circuit intégré contient un Field Programmable Gate Array (FPGA).

10. Appareil d'analyse automatique ayant un système (1) de mesure de transmission suivant l'une des revendications 6 à 9.
